# EUROPEAN PATENT SPECIFICATION

(11) **EP 0 662 540 B1**
(45) Date of publication and mention of the grant of the patent: **12.03.1997**
(21) Application number: 94120066.9
(22) Date of filing: 19.12.1994
(51) Int. Cl.: D06M 16/00, D06M 101/28

(54) **Process for the surface modification of solid polyacrylonitrile articles**
Verfahren zur Oberflächenmodifizierung von festen Polyacrylnitril-Artikeln
Procédé pour la modification en surface d'articles solides en polyacrylonitrile

(30) Priority: 11.01.1994 IT MI940016
(43) Date of publication of application: 12.07.1995
(73) Proprietor: Montefibre S.p.A., Milan (IT)
(72) Inventor: Battistel, Ezio, I-28100 Novara (IT); Francalanci, Franco, I-28100 Novara (IT); Marinetti, Massimo, I- Mestre (IT); Morra, Marco, I-14040 Cartiglione d'Asti (IT)
(74) Representative: Fusina, Gerolamo

(56) References cited:
- EP-A- 0 371 258
- EP-A- 0 397 119
- FR-A- 2 294 999

## Description

The present invention relates to a process for the surface modification of solid polyacrylonitrile articles.

More specifically the invention relates to a process for the surface modification of acrylic and modacrylic polymer particles or products obtained therefrom, such as fibres, woven or non-woven fabrics, based on treatment with enzymes.

An acrylic polymer means a linear polymer having at least 85% by weight of acrylonitrile in the chain.

A modacrylic polymer is a linear polymer having at least 50% but not more than 85% by weight of acrylonitrile and quantities varying from 15% to 50% by weight of vinylidene chloride or bromide in the chain.

An acrylic or modacrylic fibre is a fibre obtained starting from an acrylic polymer or modacrylic polymer respectively by the dissolution of this in a suitable solvent and subsequent dry or wet spinning as is known in the art.

The materials thus treated have greater hydrophilic properties, which improve their comfort characteristics (in practice the fabrics are softer to handle). In addition, the possibility of dyeing them with acidic dyes makes them suitable for the preparation of yarns mixed with natural fibres such as wool.

Various examples of enzymatic processes for the preparation of polymers modified on the surface are mentioned in literature. These however refer only to natural polymers. (Borgondo, E. Fornelli. S., 1992, Nuova Selezione Tessile, May 1992, 116).

In the case of synthetic polymers, the known methods are mainly based on the action of strong chemical reagents or physical treatments. The purpose of these treatments is to introduce new functional chemical groups or to modify the groups already present on the surface of the fibres.

For example EP-A-247975 describes the treatment of acrylic fibres with strongly reactive chemical reagents.

Some treatments cause the conversion of the -CN groups into -COOH groups, thus increasing the surface hydrophilic property of the fibres.

Another example of chemical modification for improving the hydrophilic property of the materials based on polyacrylonitrile is the transformation of the -CN groups into -COOX groups, wherein X is a cation such as K or Na.

The reaction (saponification) can take place both under heterogeneous conditions, i.e. non-dissolved polymer (Geller, A.A., et al., 1974, J. Polym. Scien. 12,2327) or under homogeneous conditions (Sanli. O. 1990 Eur. Polym. J. 26,9).

In the first case, the reaction is influenced by the overmolecular architecture of the polymer and it is therefore not easily governable.

In the second case it is necessary to use organic solvents which are capable of dissolving the polymer.

In both cases, however, both the acid and the amide are often obtained and in addition drastic reaction conditions are required together with the presence of strong concentrations of bases (for example 25% KOH).

A further example of chemical surface modification which selectively converts nitrile groups to amide groups without side reactions to imide or carboxyl groups is described in EP-A-371258. The reaction is carried out combining in suspension in a liquid non-solvent for the polyacrylonitrile or a copolymer thereof, the substrate, an alkaline catalyst and a peroxide. The reaction mixture is then heated for a time sufficient to convert up to about 15 mole percent of the total surface nitrile groups to amide groups.

Generally, however, all the chemical processes used involve the use of organic solvents and dangerous reagents (alkalies and acids) which can also create safety problems, environmental problems and difficulties for treatment of the by-products.

In addition, these processes are difficult to repeat and control (they often lead to the undesired dissolution of the fibre).

The surface modification of synthetic polymers can also be obtained by drastic physico-chemical treatments or particular techniques at high energy density.

For example, the use of radiation (gamma or X rays) or gaseous phases in partially ionized states, such as plasma (GB-A-2.105.729, 1981), belong to this group of techniques. However the problems of safety and health, which the introduction of these techniques can create in an industrial process, are enormous especially if there are considerable quantities of materials to be treated.

In addition to these, there are also problems of a technical nature.

For example, when plasma is used there is:
- the impossibility of controlling the relative quantity of -OH, -COOH or ester groups introduced;
- lack of specificity with consequent heterogeneity of the modified surface;
- the necessity of operating under vacuum (at least 1x10⁻³ torr) and consequently considerable technical problems when large quantities of material are treated.

It would therefore be of great practical importance to overcome the problems arising from the use of both chemical and physico-chemical treatments of synthetic polymers.

The development of alternative technologies, such as those for example based on the use of a biological system, either eliminates or reduces the above disadvantages. These systems, in fact, generally have the following advantages:
- it is possible to operate in an aqueous medium under moderate conditions of both temperature (not higher than 30°C) and pH (approximately neutral);
- specific modifications can be obtained: the use of particular enzymes or enzymatic products make it possible to convert the -CN groups to -CO-NH₂ without further hydrolysis of the amide to acid;
- highly technological equipment such as that used for treatment with plasma is not required;
- the degree of modification of the polymer, and consequently its final characteristics, can be controlled.

An example of a process based on the use of a biological system is described in FR-A-2294999. In particular, this document describes the preparation of amides by biological hydrolysis of the corresponding nitriles which, in this case, are dissolved monomers.

However, biological treatment capable of modifying the surface properties of synthetic polymers, including polyacrylonitrile has not been described so far and the application of these techniques is for the moment limited to the study of the biodegradation of synthetic polymers.

The Applicant has now found in a simple and easily applicable process for the surface modification of polyacrylonitrile articles, based on the use of a biological system.

The present invention therefore relates to a process for the surface modification of solid polyacrylonitrile articles, characterized in that the articles are treated with enzymes of the nitrile hydratase group to convert nitrile groups on the surface of the article to amide groups.

The manufactured goods obtainable by the process of the invention have a greater hydrophilic property, which improves the comfort characteristics of the weaves (they are softer to the touch) In addition, the possibility of dyeing them with acidic dyes enables the preparation of yarns mixed with natural fibres such as wool.

The process of the present invention comprises all the advantages mentioned above for the biological systems, consequently eliminating the drawbacks associated with chemical and physico-chemical systems.

Enzymes of the nitrile hydratase group are present in various micro-organisms. Brevibacterium imperiale, for example, produces them in high quantities. Some of these micro-organisms, moreover, are already used industrially for the production of commodities. (Kobayashi, M., et al., 1992, Tibtech. 10,40-2). An example is represented by the production of acrylamide starting from acrylonitrile by the Japanese company Nitto.

The strain CBS 49874 of Brevibacterium imperiale available at the international centre Centraal bureau voor Schimmelcultures, Baarn, Delft, The Netherlands, was used for the embodiment of our process.

The strain belongs to a first group of isolated Brevibacterium imperiale strains shown about fifteen years ago for the production of acrylamide (Arnoud, A., et al., 1976, Acad. Sci. Paris, 287,5713).

The enzymes produced by B. imperiale are capable of hydrolizing the nitrile group to amide and then act on the surface -CN groups of the acrylic or modacrylic polymer according to the following reaction:

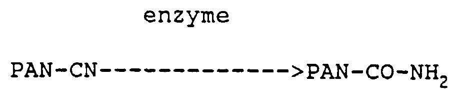

The polymeric article subjected to the reaction can be in the form of a non-spun polymer having a varying particle size, or in the form of a fibre, woven or non-woven fabric.

It can consist of pure homopolymer (100% acrylonitrile) or its synthesized copolymer starting from a mixture of up to 10% by weight of other monomers such as vinylesters (for example vinyl acetate) or acrylates, or its copolymer containing up to 50% by weight of vinylidene chloride.

The chemical modification by enzymes of the -CN groups which causes the formation of -CO-NH₂ groups is confirmed by surface analyses of the materials.

Techniques which reflect the properties of the material as a whole (such as IR, NMR) cannot be used because they are not sensitive enough to show the surface modification of the polymer, as the modified -CN groups are only a small part of the whole.

The most suitable technique is XPS (X-ray Photoelectron Spectroscopy) or ESCA (Electron Spectroscopy for Chemcial Analysis) and the apparatus used is a Perkin Elmer PHI 5500 ESCA System (X-ray source at 14 KV, 200 W).

The analysis extends for a thickness of about 6 nanometres of material.

From an examination of the data provided in the examples, the conclusion can be reached that the enzymatic modification consists in the hydrolysis of the -CN groups to amide without any further hydrolysis of the amide to carboxylic acid.

The contact angle of the fibre modified by enzymatic treatment changes with respect to the non-treated fibre.

For example, the contact angle of the whole acrylic polymer (comonomer: 5% vinyl acetate) is 53° whereas the modified one is 30°±7°.

This variation indicates a significant increase in the hydrophilic degree of the surface of the polymeric article after the enzymatic treatment.

The following examples provide a better illustration of the present invention but do not limit its scope, which is described in the enclosed claims.

### EXAMPLE 1

The enzymatic products were obtained according to the following process:

Fermentations of B. imperiale were prepared in 2-litre flasks containing 0.5 l of culture broth for a total volume of 3 l. A YMPG culture medium was used, consisting of yeast extract (3 g/l), malt extract (3 g/l), peptone (5 g/l). After sterilization of the medium, glucose was added under sterile conditions up to a final concentration of 10 g/l. The preinoculum was prepared in a YMPH medium, of which 50 ml were removed to inoculate each flask. After 72 hours of culture at 28°C, 200 rpm, the cells were centrifugated and resuspended in a 100 mM phosphate buffer, pH 7. On an average 15±3 g/l of fermentation broth of cells (humid weight) were obtained. The cells were then lysated by ultrasonic treatment at 250 watts for 60 secs, for 7 break cycles with intervals for cooling periods. The cellular lysis was spectrophotometrically controlled, by a measurement at 260 nm of the nucleic acids present in the surnatant liquid, after centrifugation of the lysis.

The test to quantitatively check the activity of the nitrile hydratase enzyme is carried out under the following conditions:
phosphate 100 mM, pH 7 (1 ml), sample (10 - 50 µl), acrylonitrile (5 µl), temperature 30°C. (Hjort, C.M., et al., 1990, Tech. Biotechnol., 48,217).

The formation of the hydrolysis product, acrylamide, was followed spectrophotometrically at 235 nm as a funtion of time.

The concentration of the hydrolysis product was calculated using an extinction coefficient of 1106 cm⁻¹1M⁻¹.

The unit of enzymatic activity is defined as the quantity of enzyme capable of transforming 1 µmole of substrate per minute at 30°C.

On an average 6±2x10⁵ U were obtained per litre of lysated cells.

### EXAMPLE 2

For the treatment of the polymer enzymatic products were used consisting of whole or partially purified cellular lysates obtained from standard B. imperiale cultures (YMPH medium, pH 7, 28°C).

The cells were lysated by ultrasonic treatment and the lysate, consisting of a turbid suspension of drift and cellular organelles, was used as such or centrifuged. In the latter case, the precipitated product was disposed of. The centrifugation causes a partial loss of the enzymatic activity.

For the enzymatic modification tests the polymer with a different particle size was used, for example with an average particle diameter of between 10 and 200 µm. To facilitate the interaction between the enzyme (soluble in a buffer solution) and polymer (insoluble in water) in some cases 2% v/v of dimethylformamide (DMF) or dimethylacetamide (DMAC) was added. DMF and DMAC are solvents which are capable of dissolving the polymer. To ensure that the presence of proteins or nucleic acids possibly non-specifically absorbed by the polymer cannot interfere with the analysis of the surface modification of the polymer, the reference polymer was treated in the same way as the samples, i.e. it was put in contact, for the same reaction time, with the cellular lysate or surnatant, after deactivating however the enzymatic activity present by treatment at 35°C for 24 hours.

The granular polymer was left to decant and the surnatant liquid eliminated.

Similarly three washing cycles with water were repeated, centrifuging each time at 3-4000 rpm for 2-3 minutes and the surnatant liquid was disposed of. The polymer was then filtered on Gooch washed with 50% ethanol (3 times), water (3 times) and finally acetone (3 times).

The test samples were then dried under vacuum (with a water pump). The fibre and weave were treated in the same way, without centrifugation.

The following test samples were then treated as described in detail in examples 3 - 7.
1. acrylic polymer treated with whole cellular lysate
3. acrylic polymer treated with whole cellular lysate + 2% DMF.
2. acrylic polymer treated with surnatant liquid of the centrifuged lysate.
4. acrylic polymer treated with surnatant liquid of the centrifuged lysate + 2% DMF
7. acrylic polymer as such (treated with whole deactivated cellular lysate)
8. acrylic polymer as such (treated with inactivated surnatant of the centrifuged lysate)
   After 24 hours the samples were washed and analyzed to show that the enzymatic attack had taken place.
   At the same time samples of spun polymer (fiber) and woven were also prepared. The average diameter of the fibers can be between 2-30 µm.
5. acrylic fiber treated with whole lysate
6. acrylic fiber treated with whole lysate + 2% DMF
9. acrylic fiber treated with whole deactivated lysate
10. fabric as such
11. fabric treated with whole cellular lysate To show the surface modification of the polymer, of the fiber and of the fabric, the XPS (X-ray Photoelectron Spectroscopy) or ESCA (Electron Spectroscopy for Chemical Analysis) technique was used.

The analysis is extended for a thickness of about 6 nanometers of the material.

The results of the XPS analysis on the polymer, fiber and woven samples prepared as described, are shown in Table 1.

The results refer to the relative percentages of Oxygen, Nitrogen and Carbon present on the surface of the samples.

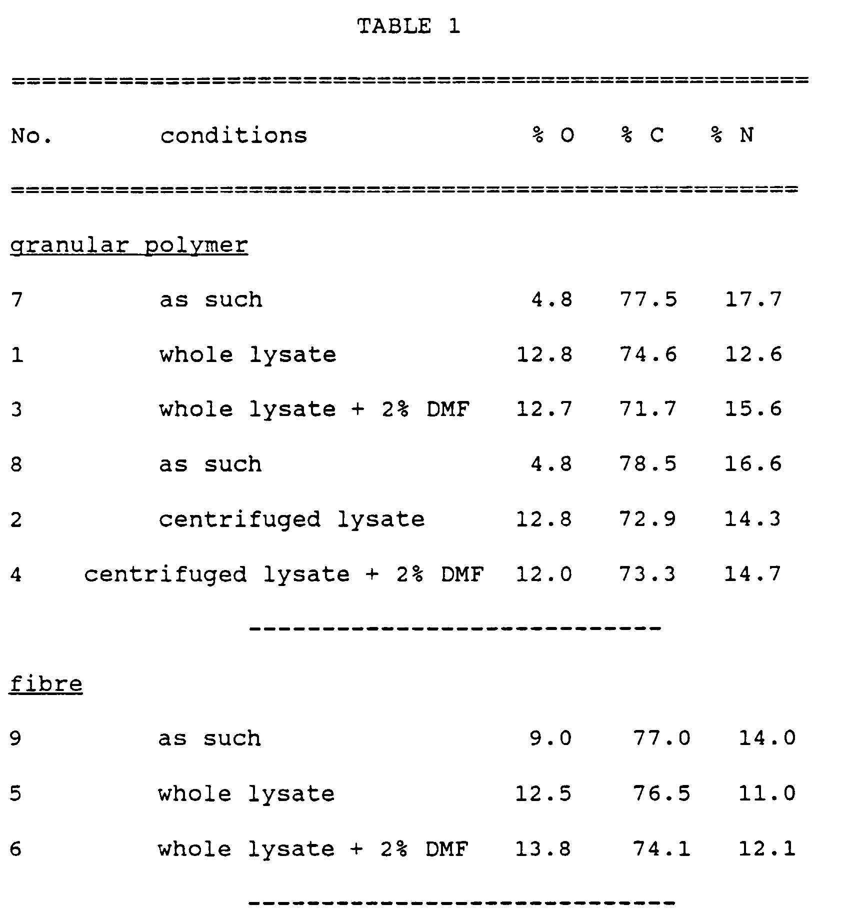

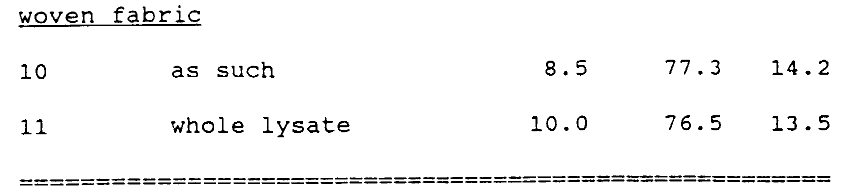

As can be seen, the content oxygen present on the surface of the polymeric particles, fibers and wovens significantly increases, as can be expected when -CN groups are transformed into amidic groups.

At the same time, the relative percentage of -CN decreases. The O/C ratio increases indicating a significant hydrophilization of the surface.

It should also be noted that the same effects are obtained both with the whole cellular lysate, richer in proteins and nitrile hydratase enzymatic activity (12.000 Units/ml), and with the surnatant of the centrifuged lysate, poorer in nitrile hydratase enzymatic activity (about 650 Units/ml).

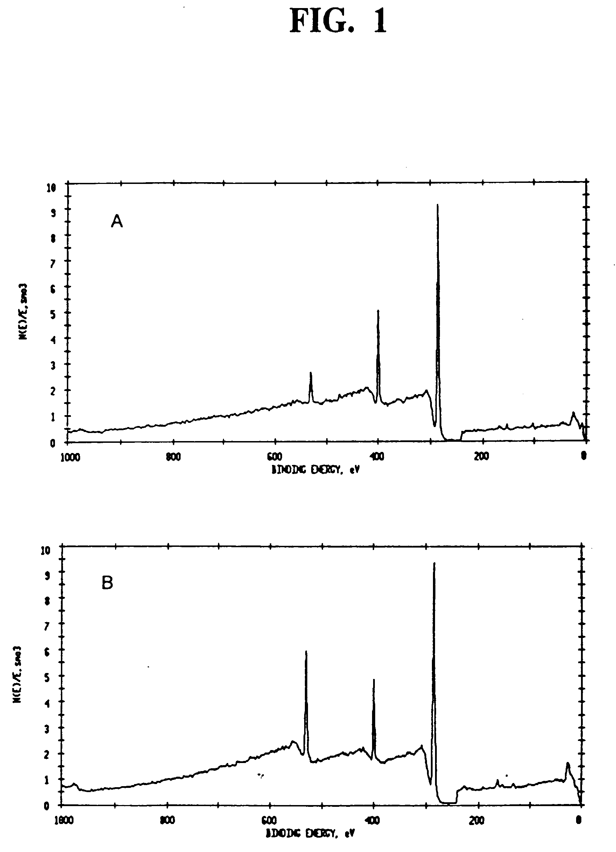

Fig. 1 shows the XPS spectrum of the polymer as such (A) and treated polymer (B) (sample 1). The peak around 530 eV refers to the oxygen, whereas those around 395 and 290 are of the nitrogen and carbon, respectively.

Integration of the peaks gives the relative percentages of the atoms present on the surface.

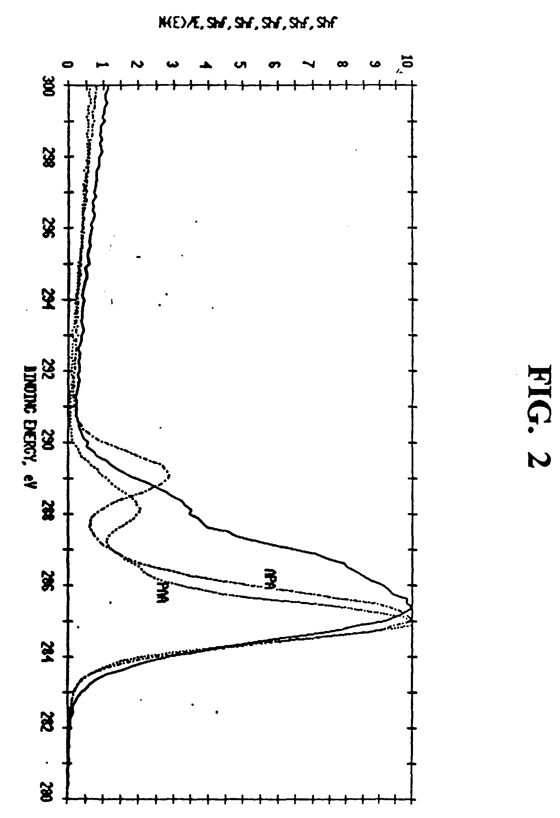

Fig. 2 shows the XPS peak of carbon of the granular acrylic polymer (continuous line) which covers those obtained from the analysis of the polyacrylic acid (APA) and polyacrylamide (PAA dotted line).

As can be seen, in the treated polymer there is a high energy bond band, around 285 eV, typical of the amidic group. In fact this band is characteristic of the PAA spectrum.

There is no component on the other hand with higher energies typical of the carboxylic group of APA. It can be concluded that the enzymatic modification consists in the hydrolysis of the -CN groups to amide without further hydrolysis of the amide to carboxylic acid.

### EXAMPLE 3

1 g of granular acrylic polymer (average particle size 30 µm) was put in contact (in a 100 ml flask) with 50 ml of B. imperiale cellular lysate in a 100 mM phosphate buffer, pH 7, (or surnatant after centrifugation) having an activity of about 8.000-12.000 U/ml (or in the case of the surnatant 400-700 U/ml). The suspension was vigorously stirred at 200-250 rpm. The temperature was maintained at 20±1°C. After 24 hours the polymer was decanted and washed, after disposing of the surnatant. The results of the XPS analysis are shown in Table 1 sample 1, and sample 2 in the case of reaction with surnatant.

### EXAMPLE 4

1 g of granular acrylic polymer (average particle size 45 µm) was reacted (in a 100 ml flask) with 50 ml of B. imperiale cellular lysate in a 100 mM phosphate buffer, pH 7, (or surnatant after centrifugation) having an activity of about 8.000-12.000 U/ml (or in the case of the surnatant 400-700 U/ml). The suspension was vigorously stirred at 200-250 rpm for 24 hours. The temperature was maintained at 20±1°C. The results of the XPS analysis are very similar to those shown in Example 3.

### EXAMPLE 5

1 g of granular acrylic polymer (average particle size 30 µm) was reacted (in a 100 ml flask) with 50 ml of B. imperiale cellular lysate in a 100 mM phosphate buffer, pH 7, (or surnatant after centrifugation) having an activity of about 8.000-12.000 U/ml (or in the case of the surnatant 400-700 U/ml). 1 ml of dimethylformamide or dimethylacetamide (2% v/v) was added to the mixture. The suspension was vigorously stirred at 200-250 rpm. The temperature was maintained at 20±1°C. The results of the XPS analysis are shown in Table 1 sample 3, and sample 4 (in the case of reaction with the surnatant).

### EXAMPLE 6

500 mg of acrylic fiber (average diameter of the microfibers 5 µm) were put in contact (in a 500 ml flask) with 30 ml of B. imperiale cellular lysate in a 100 mM phosphate buffer, pH 7, (or surnatant after centrifugation) having an activity of about 8.000-12.000 U/ml (or in the case of the surnatant 400-700 U/ml). The mixture was vigorously stirred at 200-250 rpm. The temperature was maintained at 20±1°C. After 24 hours the fiber was washed as described above. The results of the XPS analysis are shown in Table 1 sample 5, and sample 6 (in the case of reaction with surnatant).

### EXAMPLE 7

A piece of plain knitted Jersey having a weight of 20 g/m² (20x20 cm) was put in contact (in a 500 ml flask) with 50 ml of B. imperiale cellular lysate in a 100 mM phosphate buffer, pH 7 having an activity of about 8.000-12.000 U/ml After 24 hours of vigorous stirring (200-250) at 20±1°C, the piece of weave was washed. The results of the XPS analysis are shown in Table 1, sample 11.

### EXAMPLE 8

1 gram of granular acrylic polymer (average particle size 45 µm) treated with cellular lysate from Brevibacterium imperiale (as described in example 2) and 1 gram of standard acrylic polymer (not enzymatically treated) were put in 2 hermetic flask each containing 50 ml of a solution (100 mg/l) of Polar Red B-01 Ciba, acidified with sulphuric acid at pH 2.1. The two samples were put in an oven for 60 min. at 98°C. After recovering the surnatant liquid, the samples were filtered on gooch and washed four times with water.

The standard polymer maintained the same starting colour (white), whereas the enzymatically treated polymer had become dark red.

The conditions used for the dyeing (pH, temperature and duration of treatment) are similar to those normally used for the dyeing of wool.

## Claims

1. A process for the surface modification of solid polyacrylonitrile articles, characterized in that the articles are treated with enzymes of the nitrile hydratase group to convert nitrile groups on the surface of the article to amide groups.

2. Process according to claim 1 characterized in that the polyacrylonitrile articles are treated with products containing nitrile hydratase enzymes consisting of whole or partially purified cellular lysates of micro-organisms of the species Brevibacterium.

3. Process according to claim 2 characterized in that the polyacrylonitrile articles are treated with preparations containing nitrile hydratase enzymes of the strain Brevibacterium imperiale CBS 49874 available at the international centre Centraalbureau voor Schimmelcultures, Baarn, Delft, The Netherlands.

4. Process according to any of the previous claims from 1 to 3 characterized in that the transformation reaction of the nitrile group into amidic group is carried out under vigorous stirring in the presence of 2000-12000 U/ml of enzyme, 0.2-2 g of polymer, in a medium consisting of a 100 mM phosphate buffer, pH 7, at temperatures of between 10 and 30°C, for 12-24 hours.

5. Process according to claim 4 characterized in that the reaction medium also comprises dimethylformaldehyde (DMF) at concentrations of between 1 and 3% v/v.

6. Process according to any of the claims from 1 to 5 characterized in that the polyacrylonitrile articles which are subjected to treatment with enzymes are in the form of a non-yarn polymer having a varying particle size and average particle diameter of between 10 and 200 µm.

7. Process according to any of the claims from 1 to 5 characterized in that the polyacrylonitrile articles which are subjected to treatment with enzymes are in the form of yarn polymer, fiber or weave, and the average diameter of the fibers is between 2 and 30 µm.

8. Process according to any of the claims from 1 to 5 characterized in that the polyacrylonitrile articles which are subjected to treatment with enzymes are homopolymers or copolymers, where the comonomer is a vinylester or an acrylate.

## Patentansprüche

1. Verfahren zur Oberflächenmodifizierung von festen Polyacrylnitril-Gegenständen,
dadurch **gekennzeichnet,** daß
die Gegenstände mit Enzymen aus der Nitrilhydratase-Gruppe behandelt werden, zum Umwandeln von Nitril-Gruppen auf der Oberfläche des Gegenstandes in Amid-Gruppen.

2. Verfahren nach Anspruch 1,
dadurch **gekennzeichnet,** daß
die Polyacrylnitril-Gegenstände mit Produkten behandelt werden, umfassend Nitrilhydratase-Enzyme, die aus insgesamt oder teilweise gereinigten zellulären Lysaten von Mikroorganismen der Spezies Brevibacterium bestehen.

3. Verfahren nach Anspruch 2,
dadurch **gekennzeichnet,** daß
die Polyacrylnitril-Gegenstände mit Präparaten behandelt werden, umfassend Nitrilhydratase-Enzyme des Stammes Brevibaceterium imperiale CBS 49874, erhältlich bei dem internationalen Zentrum Centraalbureau voor Schimmelcultures, Baarn, Delft, Niederlande.

4. Verfahren nach einem der vorhergehenden Ansprüche 1 bis 3,
dadurch **gekennzeichnet,** daß
die Transformationsreaktion der Nitril-Gruppen in Amid-Gruppen unter heftigem Rühren in der Gegenwart von 2000 - 12000 U/ml Enzym, 0,2 - 2 g Polymer in einem Medium, bestehend aus 100 mM Phosphatpuffer, pH 7, bei Temperaturen zwischen 10 und 30°C für 12 bis 24 h durchgeführt wird.

5. Verfahren nach Anspruch 4,
dadurch **gekennzeichnet,** daß
das Reaktionsmedium ebenfalls Dimethylformaldehyd (DMF) bei Konzentrationen zwischen 1 und 3 % v/v enthält.

6. Verfahren nach einem der Ansprüche 1 bis 5,
dadurch **gekennzeichnet,** daß
die Polyacrylnitril-Gegenstände, die der Behandlung mit Enzymen unterworfen werden, in der Form eines Nicht-Garn-Polymers mit variierender Teilchengröße und einem durchschnittlichen Teilchendurchmesser zwischen 10 und 200 µm sind.

7. Verfahren nach einem der Ansprüche 1 bis 5,
dadurch **gekennzeichnet,** daß
die Polyacrylnitril-Gegenstände, die der Behandlung mit Enzymen unterworfen werden, in der Form eines Garn-Polymers, einer Faser oder eines Gewebes vorliegen und daß der durchschnittliche Durchmesser der Fasern zwischen 2 und 30 µm ist.

8. Verfahren nach einem der Ansprüche 1 bis 5,
dadurch **gekennzeichnet,** daß
die Polyacrylnitril-Gegenstände, die der Behandlung mit Enzymen unterworfen werden, Homopolymere oder Copolymere sind, worin das Comonomer ein Vinylester oder ein Acrylat ist.

## Revendications

1. Procédé qui consiste à modifier la surface d'articles solides en polyacrylonitrile, caractérisé en ce que l'on traite les articles avec des enzymes appartenant au groupe des nitrile-hydratases pour transformer les groupes nitrile à la surface de l'article en groupes amide.

2. Procédé conforme à la revendication 1, caractérisé en ce que l'on traite les articles en polyacrylonitrile avec des produits contenant des enzymes de type nitrile-hydratase et comprenant des lysats cellulaires de micro-organismes des espèces Brevibactérium partiellement ou entièrement purifiés.

3. Procédé conforme à la revendication 2, dans lequel on traite les articles en polyacrylonitrile avec des préparations contenant des enzymes de type nitrile-hydratase de la souche Brevibactérium impériale CBS 49874 disponible au centre international Centraalbureau voor Schimmelcultures, Baarn, Delft, Pays Bas.

4. Procédé conforme à l'une quelconque des précédentes revendications 1 à 3, caractérisé en ce que l'on effectue la réaction de transformation des groupes nitrile en groupe amide sous une agitation énergique en présence de 2000 à 12000 U/ml d'enzyme, de 0,2 à 2 g de polymère, dans un milieu comprenant un tampon phosphate 100 mM, pH 7, à des températures comprises entre 10 et 30° C pendant 12 à 24 heures.

5. Procédé conforme à la revendication 4, caractérisé en ce que le milieu réactionnel comprend également le diméthylformaldéhyde (DMF) à des concentrations comprises entre 1 et 3 % en volumes.

6. Procédé conforme à l'une quelconque des revendications 1 à 5, caractérisé en ce que les articles en polyacrylonitrile, que l'on soumet à un traitement avec des enzymes, sont sous la forme d'un polymère non-filé présentant différentes tailles de particule et un diamètre moyen de particule compris entre 10 et 200 µm.

7. Procédé conforme à l'une quelconque des revendications 1 à 5, caractérisé en ce que les articles en polyacrylonitrile, que l'on soumet à un traitement avec des enzymes, sont sous la forme d'un polymère filé, d'une fibre ou d'un tissage, et le diamètre moyen des fibres est compris entre 2 et 30 µm.

8. Procédé conforme à l'une quelconque des revendications 1 à 5, caractérisé en ce que les articles en polyacrylonitrile, que l'on soumet à un traitement avec des enzymes, sont des homopolymères ou des copolymères, où le comonomère est un vinylester ou un acrylate.
